# EUROPEAN PATENT APPLICATION

(11) **EP 4 268 736 A1**
(43) Date of publication of application: **01.11.2023**
(21) Application number: 21915717.9
(22) Date of filing: 27.12.2021
(51) Int. Cl.: A61B 17/3205, A61B 17/32, A61F 5/00, A61B 17/072, A61B 17/11, A61B 17/34

(54) **SLEEVE GASTRECTOMY AID DEVICE**

(30) Priority: 28.12.2020 KR 20200184776
(71) Applicant: Ajou University Industry-Academic Cooperation Foundation, Gyeonggi-do 16499 (KR); Soonchunhyang University Industry Academy Cooperation Foundation, Chungcheongnam-do 31538 (KR)
(72) Inventor: LEE, Moon Gu, Suwon-si, Gyeonggi-do 16499 (KR); KIM, Sang Hyun, Seoul 05287 (KR); YUN, Sang Chul, Seoul 04069 (KR); CHO, Sung Woo, Seoul 05502 (KR); JUNG, Chang Ho, Seoul 05698 (KR); KANG, Young Jae, Gwangju 62250 (KR)
(74) Representative: Zacco Sweden AB
(86) International application number: PCT/KR2021/019929
(87) International publication number: WO 2022/145919

(57) **Abstract**

The present disclosure relates to a sleeve gastrectomy aid device including a guide configured to be inserted into a stomach of a patient; a plurality of expanders configured to be disposed in a longitudinal direction of the guide and have a volume that changes according to an amount of air therein; a sealer configured to be installed at the guide and seal the plurality of expanders with each other, an injector configured to be connected to the sealer and inject air into the expander, and a measurer configured to be disposed inside the expander and measure a pressure value of the expander.

## Description

### [Technical Field]

This application claims priority to and the benefit of Korean Patent Application No. 10-2020-0184776, filed on December 28, 2020, the disclosure of which is incorporated herein by reference in its entirety.

The present disclosure relates to a sleeve gastrectomy aid device, and more particularly, to a sleeve gastrectomy aid device inserted into the stomach to aid resection surgery.

### [Background Art]

Generally, sleeve gastrectomy is a typical surgical method for severe obesity. Sleeve gastrectomy is a laparoscopic surgery in which the stomach is cut using special tools such as a camera for observation, a stapler, and a traction device, leaving only a thin, constant-diameter stomach with a volume of 100 to 150 ml. Unlike other surgeries, sleeve gastrectomy is surgery to simply reduce the size of the stomach and directly accesses the internal organs without an external incision, thus allowing a quick post-surgery recovery. As compared to other surgical methods, sleeve gastrectomy has advantages that the user's burden is low and the complication rate is the lowest. Sleeve gastrectomy is performed by inserting a bougie into the lumen of the stomach through the mouth and cutting the stomach using a stapler and a traction device according to the form of the bougie.

However, conventional surgical tools for sleeve gastrectomy do not have a means to quantitatively provide necessary information during resection, and thus the process of cutting and pulling the stomach depends on the user's skill without a set standard, and there is a problem that there is a large difference between the form of the stomach remaining after surgery and the target form of the stomach. In a case in which the volume of the remaining stomach is too small, gastroesophageal reflux occurs as a side effect, and in a case in which the volume of the remaining stomach is too large, there is a problem that a patient may fail to reduce weight or the patient's weight may increase again.

The related art of the present disclosure is disclosed in Korean Unexamined Patent Application Publication No. 10-2020-0103489 (Date of Publication: September 2, 2020, Title of Disclosure: Inserting tube for gastric resection guides).

### [Disclosure]

### [Technical Problem]

The present disclosure is directed to providing a sleeve gastrectomy aid device capable of controlling a volume corresponding to the form of the remaining stomach.

The present disclosure is directed to providing a sleeve gastrectomy aid device capable of quantitatively providing information on a resection state.

### [Technical Solution]

The present disclosure provides a sleeve gastrectomy aid device including: a guide configured to be inserted into a stomach of a patient; a plurality of expanders configured to be disposed in a longitudinal direction of the guide and have a volume that changes according to an amount of air therein; a sealer configured to be installed at the guide and seal the plurality of expanders with each other; an injector configured to be connected to the sealer and inject air into the expander; and a measurer configured to be disposed inside the expander and measure a pressure value of the expander.

Also, the sealer may include: a plurality of sealer bodies configured to be disposed to surround the guide and have ends of the expanders fixed thereto; a first insertion portion configured to pass through the sealer body and have the guide inserted thereinto; and a second insertion portion configured to pass through the sealer body and have the injector inserted thereinto.

Also, the sealer may further include a packing configured to be disposed to surround an outer side surface of the sealer body and bring the end of the expander in close contact with the sealer body.

Also, the injector may be movably connected to the sealer, and a communication state between the injector and the expander may be changed according to a direction of movement of the injector.

Also, the injector may be provided as a plurality of injectors, and each of the injectors may separately control the volume of one of the expanders.

Also, the injector may include: an injector body configured to be slidably connected to the sealer; a communicator configured to pass through the injector body and have a communication state with the expander changed in accordance with movement of the injector body; and a stopper portion configured to extend from the injector body and come in contact with the sealer to limit a movement range of the injector body.

Also, the stopper portion may include: a first stopper configured to come in contact with one side surface of the sealer as the injector body moves a predetermined distance toward one side; and a second stopper configured to be disposed to be spaced apart from the first stopper and come in contact with the other side surface of the sealer as the injector body moves a predetermined distance toward the other side.

Also, the sleeve gastrectomy aid device may further include a monitor configured to be connected to the measurer and provide resection position information through a pressure value measured by the measurer.

Also, the resection position information may be information on a distance from the expander to a resection tool.

Also, the monitor may include: a controller configured to determine a resection position based on the pressure value measured by the measurer; and a display configured to display the information determined by the controller to an outside.

Also, the controller may determine resection position sections according to a size of the pressure value measured by the measurer.

Also, the resection position sections may include: an approach-necessary section indicating a case in which the size of the pressure measured by the measurer is lower than or equal to a first set pressure; a separation-necessary section indicating a case in which the size of the pressure measured by the measurer is higher than or equal to a second set pressure; and a resection-possible section indicating that the size of the pressure measured by the measurer is between the first set pressure and the second set pressure.

Also, the display may display different colors according to the resection position sections.

Also, the measurer may be provided as a plurality of measurers, and each of the measurers may independently measure a pressure applied to one of the expanders.

Also, the controller may compare pressure values measured by the plurality of measurers to determine whether the resection tool is being incorrectly operated.

Also, the controller may determine whether a difference between the pressure values measured by any one pair of measurers among the plurality of measurers exceeds a set size.

In addition, in a case in which the difference between the pressure values measured by any one pair of measurers is the set size or more, the display may generate a warning signal.

### [Advantageous Effects]

In a sleeve gastrectomy aid device according to the present disclosure, since a guide is provided so that its form can be freely changed, insertion of the guide into the stomach can be smoothly performed.

Also, in the sleeve gastrectomy aid device according to the present disclosure, since volumes of a plurality of expanders can be separately controlled by a plurality of injectors, application to patients with stomachs of different forms and sizes is possible.

Also, in the sleeve gastrectomy aid device according to the present disclosure, since a controller provides quantitative information through pressure inside the stomach that is measured by a measurer, an operator can form the remaining stomach in a consistent form regardless of the operator's skill or sense.

In addition, in the sleeve gastrectomy aid device according to the present disclosure, since a display displays different colors according to a resection position section, resection position information and resection length information can be provided to be more intuitively identifiable.

### [Description of Drawings]

FIG. 1 is an installation state diagram illustrating an installation state of a sleeve gastrectomy aid device according to one embodiment of the present disclosure.
FIGS. 2 and 3 are perspective views illustrating installation states of the sleeve gastrectomy aid device according to one embodiment of the present disclosure.
FIG. 4 is a lateral view schematically illustrating a configuration of the sleeve gastrectomy aid device according to one embodiment of the present disclosure.
FIG. 5 is an exploded perspective view schematically illustrating the configuration of the sleeve gastrectomy aid device according to one embodiment of the present disclosure.
FIG. 6 is a perspective view schematically illustrating a configuration of a sealer according to one embodiment of the present disclosure.
FIG. 7 is a perspective view schematically illustrating a configuration of an injector according to one embodiment of the present disclosure.
FIGS. 8A and 8B are cross-sectional views schematically illustrating operation states of the injector according to one embodiment of the present disclosure.
FIGS. 9A and 9B are perspective views schematically illustrating the operation states of the injector according to one embodiment of the present disclosure.
FIGS. 10 and 11 are perspective views schematically illustrating a configuration of the injector according to another embodiment of the present disclosure.
FIG. 12 is a lateral view schematically illustrating the configuration of the injector according to another embodiment of the present disclosure.
FIGS. 13A and 13B are operation diagrams schematically illustrating operation states of the injector according to another embodiment of the present disclosure.
FIG. 14 is a flowchart schematically illustrating a configuration of a monitor according to one embodiment of the present disclosure.
FIG. 15 is a view schematically illustrating resection position sections determined by a controller according to one embodiment of the present disclosure.
FIGS. 16A and 16B are operation diagrams schematically illustrating operation states of the controller in the process of inserting the guide.
FIGS. 17A and 17B are operation diagrams schematically illustrating operation states of the controller in an approach-necessary section.
FIGS. 18A and 18B are operation diagrams schematically illustrating operation states of the controller in a separation-necessary section.
FIGS. 19A and 19B are operation diagrams schematically illustrating operation states of the controller in a resection-possible section.
FIG. 20 is a view schematically illustrating a state in which a resection tool is incorrectly operated according to one embodiment of the present disclosure.
FIG. 21 is a flowchart schematically illustrating the process in which the monitor determines whether the resection tool is being incorrectly operated according to one embodiment of the present disclosure.

### [Modes of the Invention]

Hereinafter, embodiments of a sleeve gastrectomy aid device according to the present disclosure will be described with reference to the accompanying drawings.

In this process, thicknesses of lines or sizes of elements illustrated in the drawings may be exaggerated for clarity and convenience of description. Also, terms used below are terms defined in consideration of functions in the present disclosure and may vary according to an intention or customary practice of a user or an operator. Therefore, the terms should be defined based on the content throughout the specification.

Also, in this specification, when a certain portion is described as being "connected (or linked)" to another portion, this may include not only a case in which the portion is "directly connected (or linked)" to the other portion but also a case in which the portion is "indirectly connected (or linked)" to the other portion while another member is disposed therebetween. In this specification, when a certain portion is described as "including (or having)" a certain element, unless particularly described otherwise, this indicates that the certain portion may further "include (or have)" other elements instead of excluding other elements.

Also, throughout the specification, like reference numerals may refer to like elements. Even when not mentioned or described with reference to specific drawings, like or similar reference numerals may be described based on other drawings. Also, parts not denoted by reference numerals in specific drawings may be described based on other drawings. Also, the number, shape, and size of specific elements, a relative difference between the sizes of the elements, and the like included in the drawings of the present application are set for convenience of understanding and may be implemented in various other forms while not limiting the embodiments.

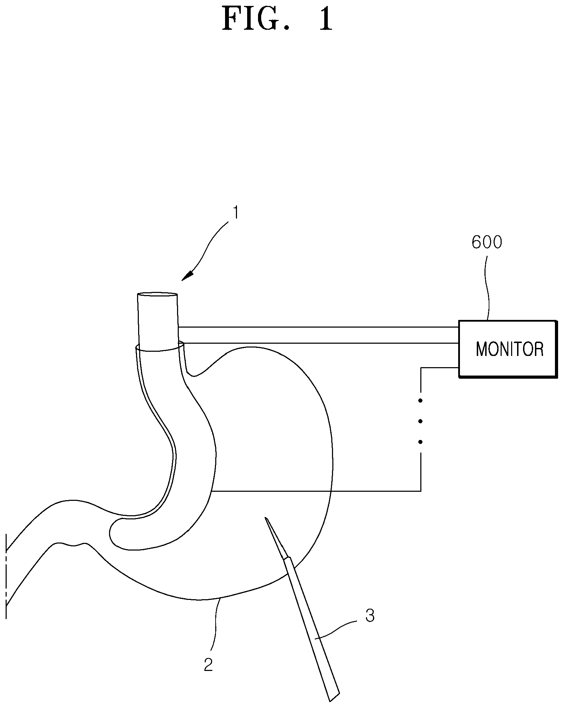

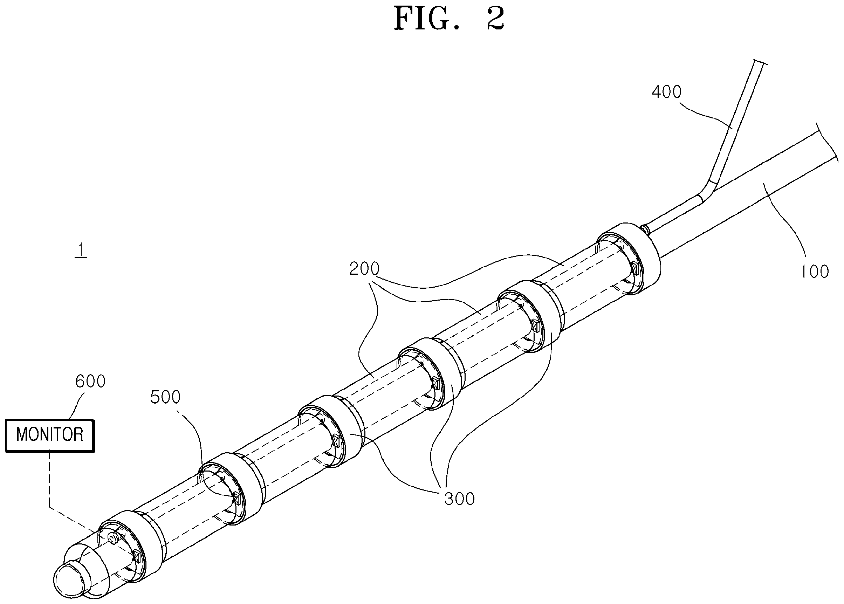

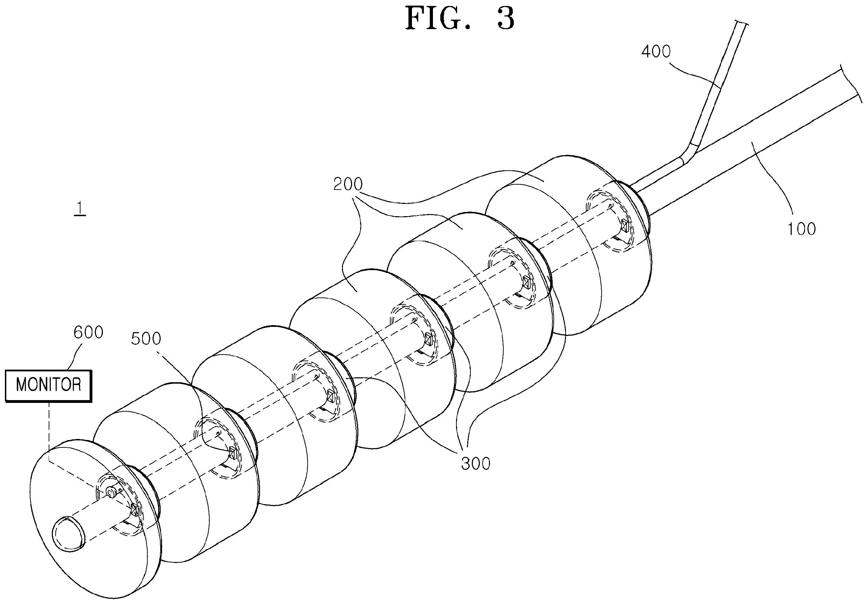

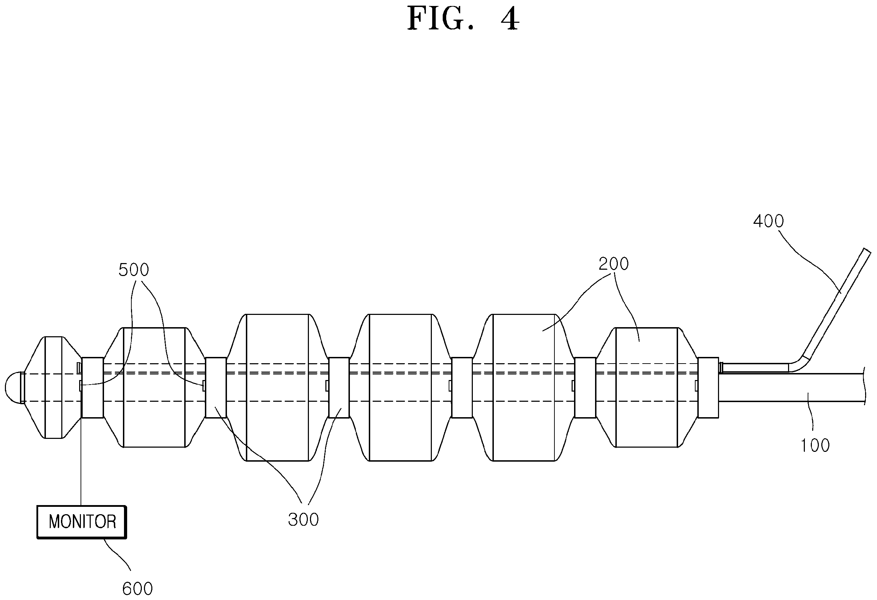

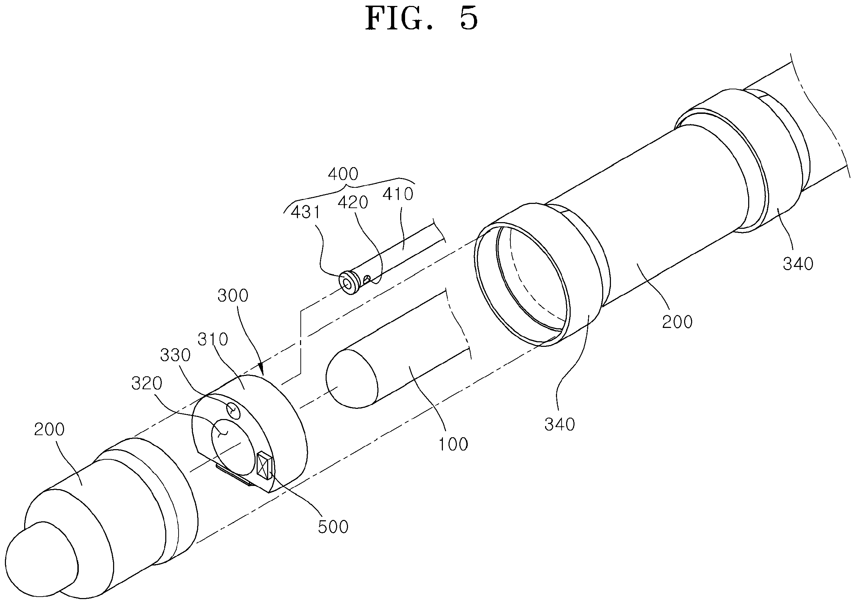

FIG. 1 is an installation state diagram illustrating an installation state of a sleeve gastrectomy aid device according to one embodiment of the present disclosure, FIGS. 2 and 3 are perspective views illustrating installation states of the sleeve gastrectomy aid device according to one embodiment of the present disclosure, FIG. 4 is a lateral view schematically illustrating a configuration of the sleeve gastrectomy aid device according to one embodiment of the present disclosure, and FIG. 5 is an exploded perspective view schematically illustrating the configuration of the sleeve gastrectomy aid device according to one embodiment of the present disclosure.

Referring to FIGS. 1 to 5, a sleeve gastrectomy aid device 1 according to one embodiment of the present disclosure includes a guide 100, an expander 200, a sealer 300, an injector 400, a measurer 500, and a monitor 600.

During sleeve gastrectomy, the guide 100 is inserted into a stomach 2 via esophagus through an oral cavity or nasal cavity of a patient. The guide 100 supports the expander 200 and the sealer 300 which will be described below and guides insertion of the expander 200 and the sealer 300 into the stomach 2. The guide 100 may include a flexible material such as silicone or synthetic resin so that the form of the guide 100 may be changed. Accordingly, the insertion of the guide 100 into the stomach 2 can be more smoothly performed, and by being bent corresponding to the flexure, form, or the like of the stomach, the guide 100 can roughly determine the form of the stomach 2 remaining after the resection surgery.

The guide 100 according to one embodiment of the present disclosure is formed to have a tubular form including a silicone material. A diameter of the guide 100 may be formed to have a smaller value than a diameter of the patient's esophagus. The length of the guide 100 may be changed to various other values within the range of the length that allows the guide 100 to be inserted into the stomach 2. An end of the guide 100 is formed to be convex with a predetermined curvature, and thus the insertion of the guide 100 into the stomach 2 can be more smoothly performed.

The expander 200 is installed outside the guide 100, and the volume of the expander 200 changes as the expander 200 expands or contracts according to the amount of air therein. As the volume of the expander 200 is controlled so that the expander 200 has a predetermined volume inside the stomach 2, the expander 200 determines the volume of the stomach 2 remaining after the resection. The expander 200 is provided as a plurality of expanders 200, and the plurality of expanders 200 are disposed to be spaced apart at predetermined intervals in the longitudinal direction of the guide. The expanders 200 are sealed with each other by the sealer 300 which will be described below. The expanders 200 may include a natural resin material such as rubber or latex having flexibility or a synthetic resin material such as chloroprene having flexibility.

The expander 200 according to one embodiment of the present disclosure is formed in the form of a cylindrical rubber membrane and disposed to surround an outer peripheral surface of the guide 100. As air is injected into the expander 200 by the injector 400 which will be described below, the volume of the expander 200 expands in a radial direction of the guide 100. The expander 200 is provided as a plurality of expanders 200, and the plurality of expanders 200 are disposed to be spaced apart at predetermined intervals in the longitudinal direction of the guide 100. In this case, the expander 200 disposed at the foremost portion is formed so that only a rear end is open, and the remaining expanders 200 excluding the expander 200 disposed at the foremost portion are formed so that both ends are open. The predetermined intervals at which the plurality of expanders 200 are spaced apart may be changed in various ways according to the size of the width or the like of the sealer 300 which will be described below. The number of expanders 200 is not limited to the number illustrated in FIGS. 1 to 4 and may be changed to various numbers according to the length or the like of the guide 100.

The sealer 300 is installed at the guide 100 and provided to seal the plurality of expanders 200 with each other. The sealer 300 provides a space in which the injector 400 and the measurer 500, which will be described below, can be installed and supports the injector 400 and the measurer 500.

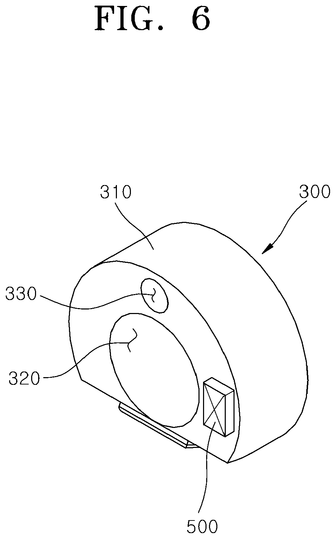

FIG. 6 is a perspective view schematically illustrating the configuration of the sealer according to one embodiment of the present disclosure.

Referring to FIGS. 2 to 6, the sealer 300 according to one embodiment of the present disclosure includes a sealer body 310, a first insertion portion 320, a second insertion portion 330, and a packing 340.

The sealer body 310 is disposed to surround an outer side surface of the guide 100 and has an end of the expander 200 fixed thereto. The sealer body 310 may be provided as a plurality of sealer bodies 310, and the plurality of sealer bodies 310 may be disposed in the longitudinal direction of the guide 100. In this case, any one sealer body 310 of the plurality of sealer bodies 310 is disposed behind the expander 200 disposed at the rearmost portion, and the remaining sealer bodies 310 are disposed between the expanders 200 neighboring each other. The end of the expander 200 may be fixed by an adhesive or the like in a state in which an inner side surface of the end is in contact with an outer side surface of the sealer body 310.

The first insertion portion 320 passes through the sealer body 310 and has the guide 100 inserted thereinto. The first insertion portion 320 according to one embodiment of the present disclosure is formed to have the form of a hole vertically passing through a central portion of the sealer body 310. A diameter of the first insertion portion 320 corresponds to the diameter of the guide 100 or is formed to have a smaller value than the diameter of the guide 100. Accordingly, the outer peripheral surface of the guide 100 inserted into the first insertion portion 320 may come in close contact with an inner peripheral surface of the sealer body 310 in which the first insertion portion 320 is formed.

The second insertion portion 330 passes through the sealer body 310 and has the injector 400, which will be described below, inserted thereinto. The second insertion portion 330 according to one embodiment of the present disclosure is formed to have the shape of a hole vertically passing through the sealer body 310. The second insertion portion 330 is disposed to be spaced a predetermined distance apart from the first insertion portion 320 in a radial direction of the sealer body 310.

The packing 340 is disposed to surround the outer side surface of the sealer body 310 and brings the end of the expander 200 in close contact with the sealer body 310. Accordingly, the packing 340 may firmly fix the expander 200 to the sealer body 310 while preventing introduction of foreign matter into the expander 200. The packing 340 according to one embodiment of the present disclosure is formed in the shape of a hollow ring. An inner side surface of the packing 340 comes in contact with the end of the expander 200 that is fixed to the outer side surface of the sealer body 310. By pressing the end of the expander 200 inward in the radial direction, the packing 340 brings the expander 200 in close contact with an inner side surface of the sealer body 310.

The injector 400 is connected to the sealer 300 and injects air into the expander 200. The injector 400 is movably connected to the sealer 300, and a communication state between the injector 400 and the expander 200 is changed according to a direction of movement of the injector 400.

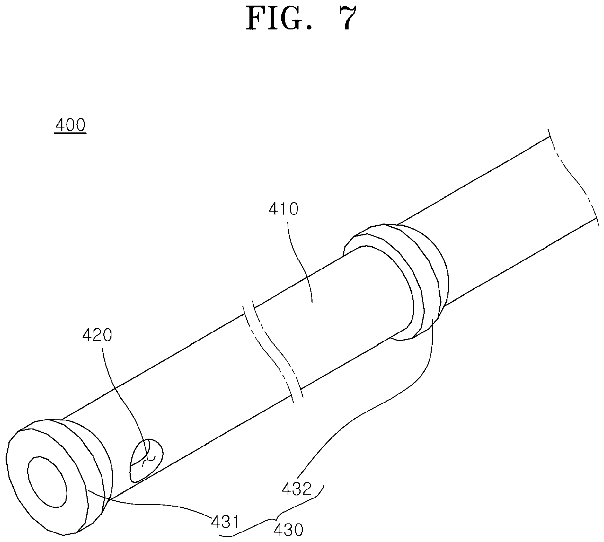

FIG. 7 is a perspective view schematically illustrating the configuration of the injector according to one embodiment of the present disclosure.

Referring to FIGS. 2 to 7, the injector 400 according to one embodiment of the present disclosure includes an injector body 410, a communicator 420, and a stopper portion 430.

The injector body 410 is slidably connected to the sealer 300. The injector body 410 has one side connected to an air supply device (not illustrated), such as an air pump, to receive air. The injector body 410 is formed to be hollow to provide a movement path for the air received from the air supply device. The injector body 410 according to one embodiment of the present disclosure is formed in the form of a hollow tube. As the injector body 410 is inserted into the second insertion portion 330 and passes through the plurality of sealer bodies 310, the injector body 410 is disposed inside the expander 200. In this case, the second insertion portions 330 formed in the plurality of sealer bodies 310 are disposed to be coaxial. Accordingly, the injector body 410 may be disposed parallel to the longitudinal direction of the guide 100. A front end of the injector body 410 is closed and disposed inside the expander 200 at the foremost portion. A rear end of the injector body 410 is open and extends to the outside of the guide 100 to be connected to the air supply device. Due to an outer side surface of the injector body 410 being slidably coupled to the inner side surface of the sealer body 310, the injector body 410 may slide back and forth in the longitudinal direction of the guide 100.

The communicator 420 passes through the injector body 410 and has a communication state with the expander 200 changed in accordance with movement of the injector body 410. The communicator 420 according to one embodiment of the present disclosure is formed in the form of a hole vertically passing through a side surface of the injector body 410. The communicator 420 is provided as a plurality of communicators 420, and the plurality of communicators 420 are spaced apart at predetermined intervals in the longitudinal direction of the injector body 410 to separately supply air to the plurality of expanders 200. The number of communicators 420 corresponds to the number of expanders 200. Each of the communicators 420 is disposed at a position at which, when the injector body 410 is completely moved to one side (the right side based on FIG. 4), the communicator 420 is disposed inside the sealer body 310 and communication between the communicator 420 and the expander 200 is blocked and, when the injector body 410 is completely moved to the other side (the left side based on FIG. 4), the communicator 420 is able to communicate with each of the expanders 200.

The stopper portion 430 extends from the injector body 410 and comes in contact with the sealer 300, more specifically, the sealer body 310, to limit a movement range of the injector body 410. The stopper portion 430 according to one embodiment of the present disclosure includes a first stopper 431 and a second stopper 432. Although the case in which the first stopper 431 and the second stopper 432 come in contact with different sealer bodies 310 will be described below as an example, the present disclosure is not limited thereto, and each of the first stopper 431 and the second stopper 432 may come in contact with one of both side surfaces of the same sealer body 310.

The first stopper 431 comes in contact with one side surface of the sealer 300 as the injector body 410 moves a predetermined distance to the one side. The first stopper 431 according to one embodiment of the present disclosure is formed in the form of a disk extending in the radial direction of the sealer body 310 and is disposed at a front end of the sealer body 310. A width of the first stopper 431 is formed to be wider than a width of the second insertion portion 330. In order to block the communication between the communicator 420 and the expander 200, the first stopper 431 comes in contact with a front surface of the sealer body 310 at the foremost portion and limits movement of the injector body 410 as the injector body 410 moves a predetermined distance to the one side (the right side based on FIG. 4).

The second stopper 432 is disposed to be spaced apart from the first stopper 431 and comes in contact with the other side surface of the sealer 300 as the injector body 410 moves a predetermined distance toward the other side. The second stopper 432 according to one embodiment of the present disclosure is formed in the form of a disk extending in the radial direction of the sealer body 310 and is disposed at a rear end of the sealer body 310. A width of the second stopper 432 is formed to be wider than the width of the second insertion portion 330. In order to allow the communicator 420 to communicate with the expander 200, the second stopper 432 comes in contact with a rear surface of the sealer body 310 at the rearmost portion and limits movement of the injector body 410 as the injector body 410 moves a predetermined distance to the other side (the left side based on FIG. 4).

Hereinafter, the operation of the injector 400 according to one embodiment of the present disclosure will be described in detail.

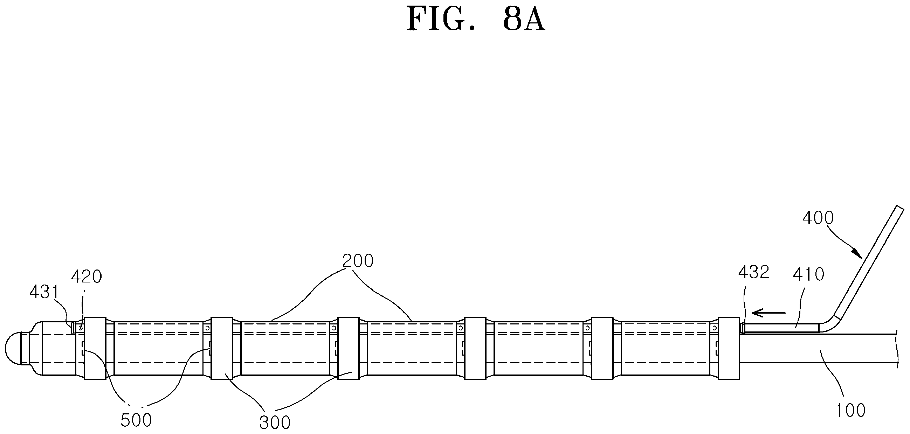

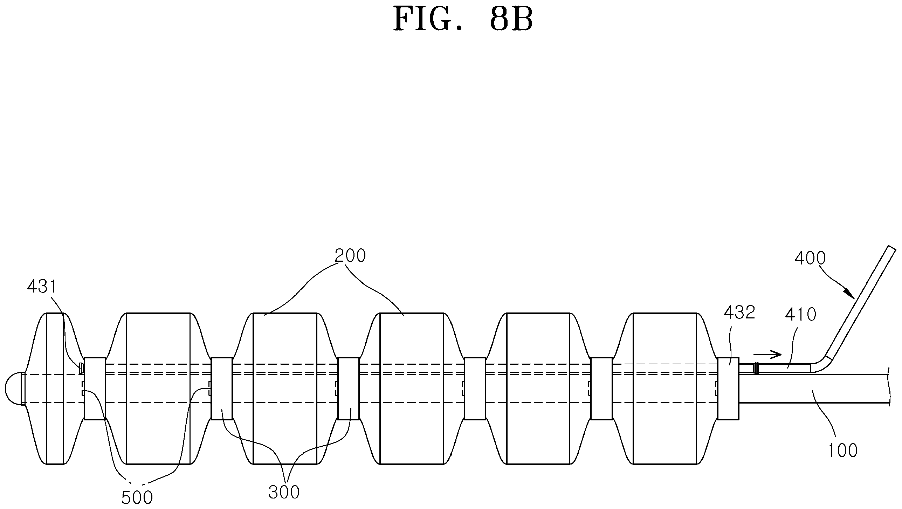

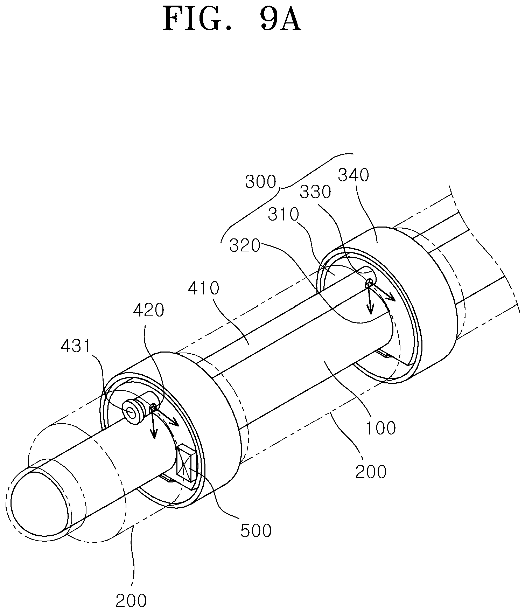

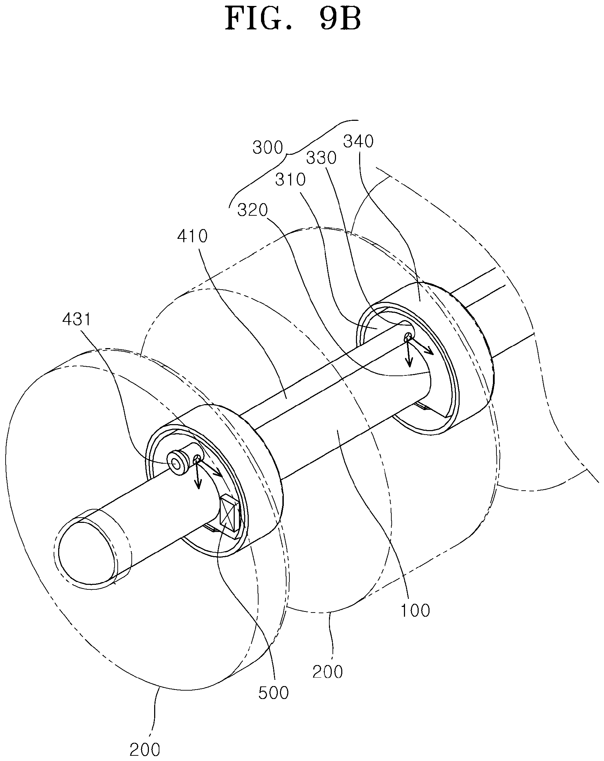

FIGS. 8A and 8B are cross-sectional views schematically illustrating operation states of the injector according to one embodiment of the present disclosure, and FIGS. 9A and 9B are perspective views schematically illustrating the operation states of the injector according to one embodiment of the present disclosure.

Referring to FIGS. 8A to 9B, the guide 100 inserted through an oral cavity or nasal cavity of a patient is inserted into the stomach 2 via the esophagus. The guide 100 inserted into the stomach 2 has an end disposed to face the pylorus of the stomach 2 and disposed close to an inner side surface of the stomach 2. In this case, the shape of the guide 100 is changed corresponding to the flexure or the like of the stomach 2.

In a state in which the guide 100 is inserted into the stomach 2, the injector body 410 is slid to the left side (based on FIG. 8A) from an initial position.

As the injector body 410 is slid a predetermined distance to the left side, the second stopper 432 comes in contact with the rear surface of the sealer body 310 disposed at the rearmost portion such that sliding of the injector body 410 stops.

Each of the communicators 420 communicates with each of the expanders 200 in accordance with the movement of the injector body 410.

Air supplied into the injector body 410 by an air injection device is injected into the expander 200 through the communicator 420.

As the amount of air inside the expander 200 gradually increases, the expander 200 expands, and the volume of the expander 200 increases in the radial direction of the guide 100.

In this case, as the end of the expander 200 is tightly fixed between the outer side surface of the sealer body 310 and the inner side surface of the packing 340 and thus air is prevented from leaking from inside the expander 200, the expander 200 may be maintained in a state in which the volume thereof is increased.

After the expansion of the expander 200 is completed, the injector body 410 is slid to the right side (based on FIG. 8B) again.

As the injector body 410 is slid a predetermined distance to the right side, the first stopper 431 comes in contact with the front surface of the sealer body 310 disposed at the foremost portion such that sliding of the injector body 410 stops.

Then, the communicator 420 is disposed to face the inner peripheral surface of the sealer body 310, and thus the communication between the communicator 420 and the expander 200 is blocked.

Hereinafter, the configuration of the injector 400 according to another embodiment of the present disclosure will be described in detail. For convenience of description, description overlapping with the above description of the injector 400 according to one embodiment of the present disclosure will be omitted.

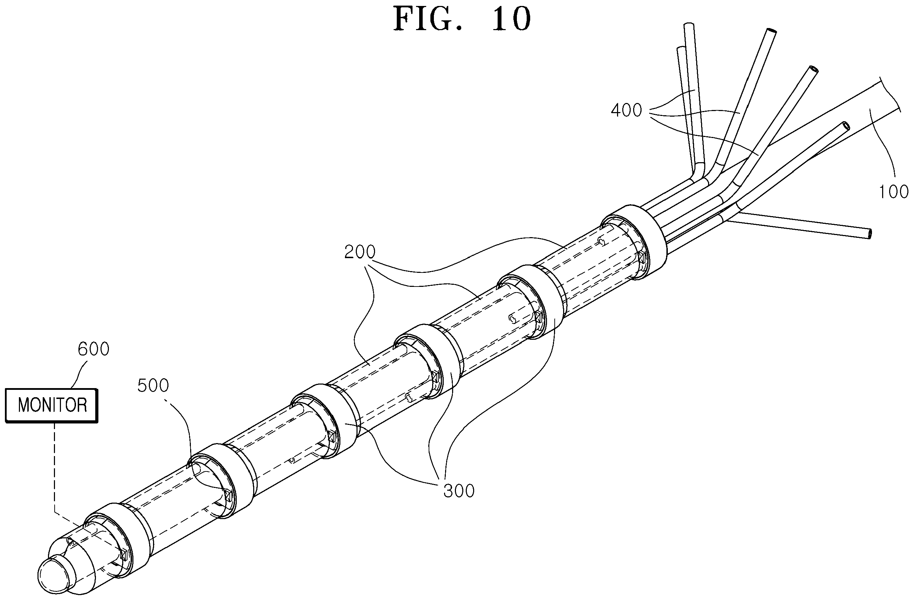

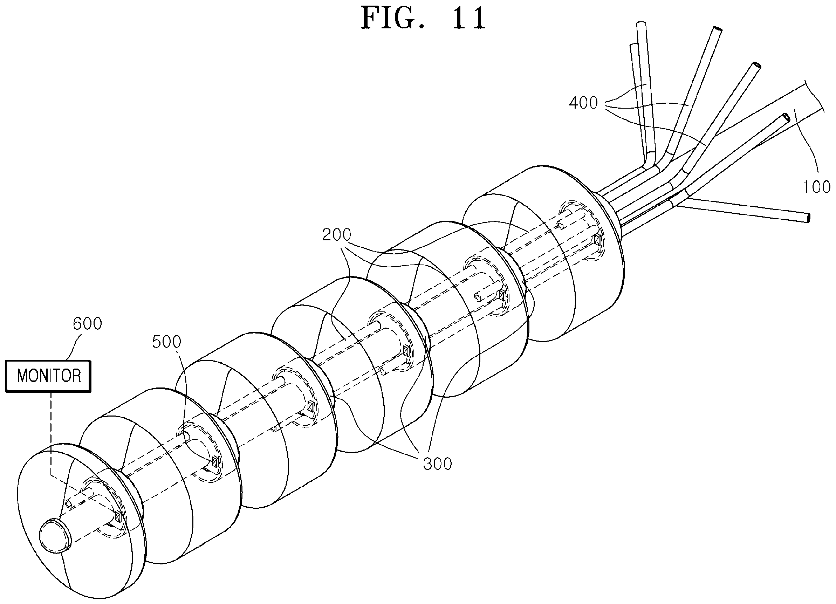

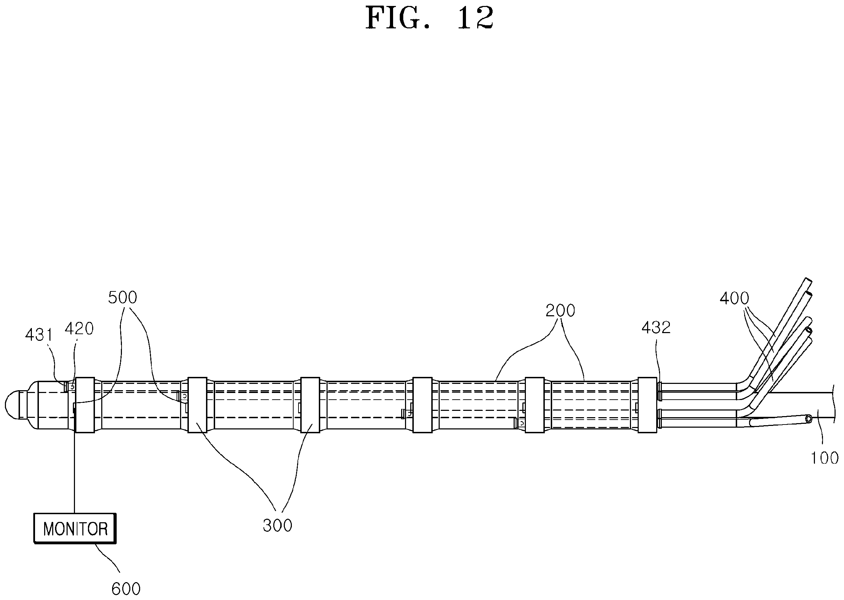

FIGS. 10 and 11 are perspective views schematically illustrating the configuration of the injector according to another embodiment of the present disclosure, and FIG. 12 is a lateral view schematically illustrating the configuration of the injector according to another embodiment of the present disclosure.

Referring to FIGS. 10 to 12, the injector 400 according to another embodiment of the present disclosure is provided as a plurality of injectors 400, and each of the plurality of injectors 400 separately controls the volume of one of the plurality of expanders 200. Accordingly, the injectors 400 may allow the expanders 200 to more accurately determine the volume of the remaining stomach 2 of the patient.

In this case, the second insertion portion 330 may be formed as a plurality of second insertion portions 330 in the sealer body 310, and the plurality of second insertion portions 330 are disposed to be spaced apart at predetermined intervals in the circumferential direction of the sealer body 310. The number of second insertion portions 330 is set differently for each sealer body 310. More specifically, in the sealer bodies 310 neighboring each other, the number of second insertion portions 330 formed in the sealer body 310 disposed at the front is one less than the number of second insertion portions 330 formed in the sealer body 310 disposed at the rear. For example, in a case in which six second insertion portions 330 are formed in the sealer body 310 disposed at the rearmost portion as illustrated in FIG. 12, five second insertion portions 330 are formed in the sealer body 310 disposed right in front of the sealer body 310 disposed at the rearmost portion. In this way, the number of second insertion portions 330 progressively decreases by one, and a single second insertion portion 330 is formed in the sealer body 310 disposed at the foremost portion. Meanwhile, the second insertion portions 330 formed at the same position of different sealer bodies 310 are disposed to be coaxial.

The injector body 410 according to another embodiment of the present disclosure is provided as a plurality of injector bodies 410. The plurality of injector bodies 410 are inserted into different second insertion portions 330 and disposed to be spaced apart in the circumferential direction of the sealer body 310. The plurality of injector bodies 410 are formed to have different lengths, and front ends of the plurality of injector bodies 410 are disposed inside different expanders 200. For example, as illustrated in FIG. 12, a front end of the injector body 410 having the longest length passes through six second insertion portions 330 and is disposed inside the expander 200 at the foremost portion. Also, a front end of the injector body 410 having the shortest length passes through only one second insertion portion 330 and is disposed inside the expander 200 at the rearmost portion. In this case, as the number of second insertion portions 330 formed in the sealer body 310 disposed at the front is one less than the number of second insertion portions 330 formed in the sealer body 310 disposed at the rear in the sealer bodies 310 neighboring each other, each of the expanders 200 may be maintained in a sealed state.

According to another embodiment of the present disclosure, a single communicator 420 is formed for each of the injector bodies 410. The communicator 420 is disposed in front of the injector body 410 protruding toward the inside of the expander 200. The communicator 420 is disposed at a position at which, when the injector body 410 is completely moved to one side (the right side based on FIG. 12), the communicator 420 is disposed inside the sealer body 310 and the communication between the communicator 420 and the expander 200 is blocked and, when the injector body 410 is completely moved to the other side (the left side based on FIG. 12), the communicator 420 is able to communicate with each of the expanders 200.

Hereinafter, the operation of the injector 400 according to another embodiment of the present disclosure will be described in detail.

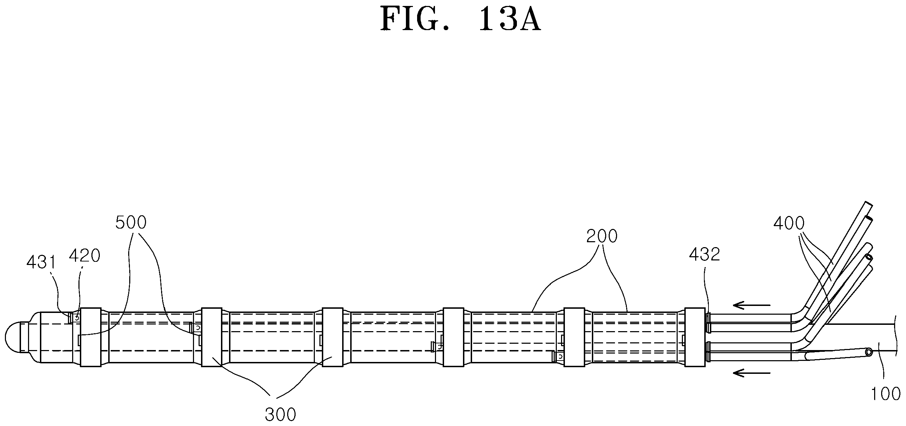

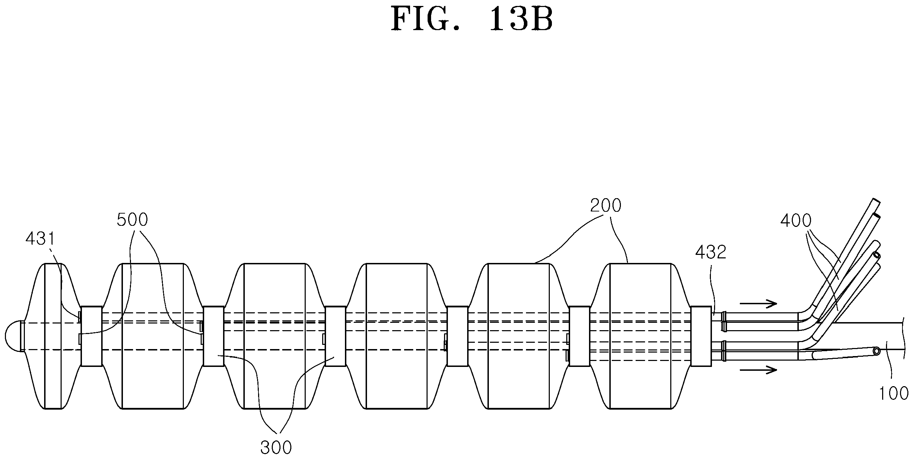

FIGS. 13A and 13B are operation diagrams schematically illustrating operation states of the injector according to another embodiment of the present disclosure.

Referring to FIGS. 13A and 13B, in a case in which the volume of any one expander 200 should be further increased, only the injector body 410 corresponding to the any one expander 200 is slid to the left side (based on FIG. 13A) from an initial position.

As the injector body 410 is slid a predetermined distance to the left side, the second stopper 432 comes in contact with the rear surface of the sealer body 310 disposed at the rearmost portion such that sliding of the injector body 410 stops.

The communicator 420 communicates with each of the expanders 200 in accordance with the movement of the injector body 410.

Air supplied into the injector body 410 by an air injection device is injected into the any one expander 200 through the communicator 420.

As the amount of air inside the expander 200 gradually increases, the any one expander 200 expands, and the volume of the any one expander 200 increases in the radial direction of the guide 100.

After the expansion of the expander 200 is completed, the injector body 410 is slid to the right side (based on FIG. 13B) again.

As the injector body 410 is slid a predetermined distance to the right side, the first stopper 431 comes in contact with the front surface of the sealer body 310 disposed at the foremost portion such that sliding of the injector body 410 stops.

Then, the communicator 420 is disposed to face the inner peripheral surface of the sealer body 310, and thus the communication between the communicator 420 and the expander 200 is blocked.

Then, in a case in which the volume of another expander 200 needs to be controlled, the above-described operation is repeated for the injector body 410 corresponding to the other expander 200.

The measurer 500 is disposed inside the expander 200 to measure a pressure value of air injected into the expander 200 and transmit the measured pressure value to the monitor 600 which will be described below. More specifically, the measurer 500 measures pressure inside the expander 200 that changes as a resection tool 3 presses the stomach 2 during the resection surgery. Due to being fixed to the sealer 300, more specifically, the sealer body 310, the measurer 500 may be disposed inside the expander 200. The measurer 500 is provided as a plurality of measurers 500, and each of the measurers 500 independently measures a pressure applied to one of the expanders 200. The measurer 500 according to one embodiment of the present disclosure may include a pressure sensor configured to measure pressure of air inside the expander 200 using a thin film dielectric method and a communication module configured to wirelessly transmit a pressure value using infrared rays, radio frequency (RF), Bluetooth, or the like.

The monitor 600 is connected to the measurer 500, determines resection position information through a pressure value measured by the measurer 500, and provides the determined resection position information to the user. Here, an example of the resection position information may be information on a distance from the expander 200 to the resection tool 3.

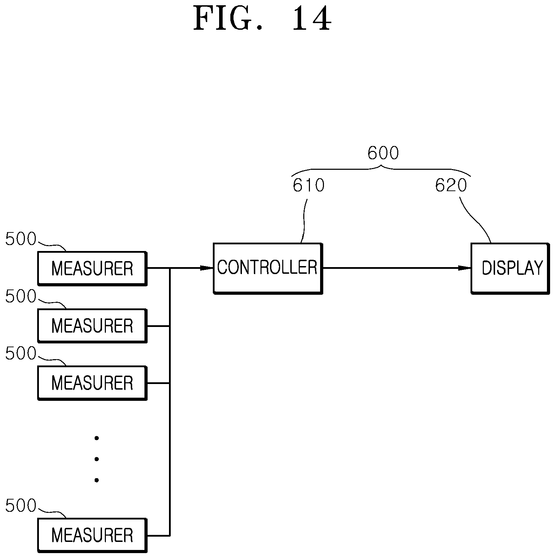

FIG. 14 is a flowchart schematically illustrating the configuration of the monitor according to one embodiment of the present disclosure.

Referring to FIG. 14, the monitor 600 according to one embodiment of the present disclosure includes a controller 610 and a display 620.

The controller 610 determines resection position information based on a pressure value measured by the measurer 500. More specifically, by determining resection position sections D according to the size of the pressure value measured by the measurer 500, the controller 610 determines the resection position information. By converting the pressure value measured by the measurer 500 into the information on the distance from the expander 200 to the resection tool 3, the controller 610 according to one embodiment of the present disclosure determines the resection position information of the resection tool 3. In this case, the controller 610 may use an average of pressure values measured by the plurality of measurers 500 to determine the resection position information of the resection tool 3.

The resection position sections D are section obtained by dividing pressure values according to the sizes thereof, based on a value of an initial pressure P0 inside the stomach 2 that is measured by the measurer 500 right after insertion of the guide 100. More specifically, the resection position sections D refer to sections between a pair of different pressure values among sections of pressures measured by the measurer 500 as the position at which the stomach 2 is pressed or resected by the resection tool 3 is changed after the insertion of the guide 100.

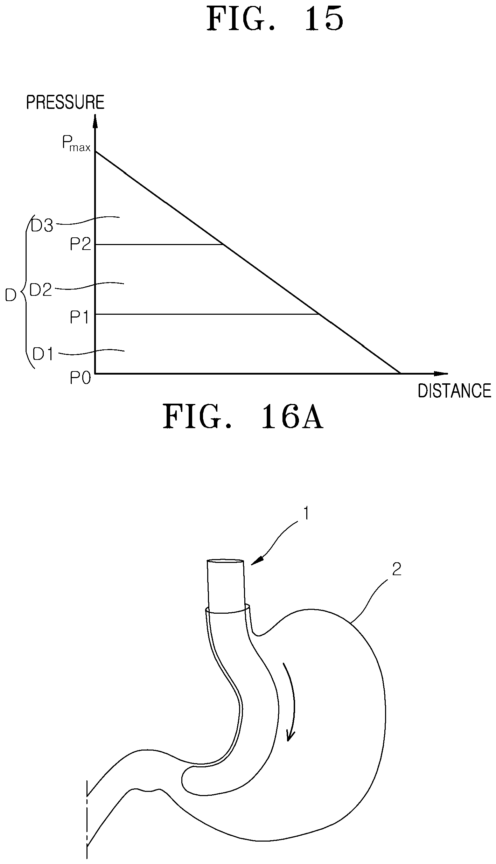

FIG. 15 is a view schematically illustrating the resection position sections determined by the controller according to one embodiment of the present disclosure.

Referring to FIG. 15, the resection position sections D according to one embodiment of the present disclosure include an approach-necessary section D1, a resection-possible section D2, and a separation-necessary section D3.

The approach-necessary section D1 indicates a case in which the size of the pressure measured by the measurer 500 is the initial pressure P0 or higher and a first set pressure P1 or lower. Here, the first set pressure P1 is a pressure value measured at the maximum distance among distances from the guide 100 to the resection tool 3 that allow the user to form the remaining stomach 2 with a target size. The first set pressure P1 may be changed in various ways according to the form, size, and the like of the stomach 2. That is, the approach-necessary section D1 corresponds to a section in which the controller 610 determines, through the pressure value measured by the measurer 500, that the resection tool 3 is excessively far away from the expander 200.

The separation-necessary section D3 indicates a case in which the size of the pressure measured by the measurer 500 is a second set pressure P2 or higher and lower than or equal to the maximum pressure that can be measured by the measurer 500 or a pressure Pmax at which rupture of mucosa of the stomach 2 occurs. Here, the second set pressure P2 is a pressure value measured at the minimum distance among the distances from the guide 100 to the resection tool 3 that allow the user to form the remaining stomach 2 with a target size. The second set pressure P2 may be changed in various ways according to the form, size, and the like of the stomach 2. That is, the separation-necessary section D3 corresponds to a section in which the controller 610 determines, through the pressure value measured by the measurer 500, that the resection tool 3 is excessively close to the expander 200.

The resection-possible section D2 indicates a case in which the size of the pressure measured by the measurer 500 is between the first set pressure P1 and the second set pressure P2. That is, the resection-possible section D2 corresponds to a section in which the controller 610 determines, through the pressure value measured by the measurer 500, that the resection tool 3 is positioned at an appropriate distance from the expander 200.

In a case in which the length of the patient's stomach is short and some of the expanders 200 are not inserted into the stomach 2, the controller 610 excludes the measurers 500 measuring pressure values of the corresponding expanders 200 from targets for determination. More specifically, the controller 610 distinguishes between the measurers 500 disposed inside the stomach 2 and measuring the same measurement value as the initial pressure P0 inside the stomach 2 and the measurers 500 disposed outside the stomach 2 and measuring a different measurement value from the initial pressure P0. The controller 610 determines that the measurers 500 measuring the same measurement value as the initial pressure P0 inside the stomach 2 are in the approach-necessary section D1 and excludes other measurers 500 from targets for determination.

The display 620 visually displays information determined by the controller 610 to the user. By a display module or the like configured to output image information, the display 620 displays different colors according to the type of resection position section D determined by the controller 610. In a case in which the measurer 500 is provided as a plurality of measurers 500, the display 620 may individually display information on each of the measurers 500.

The display 620 according to one embodiment of the present disclosure displays a first color C1 for the measurers 500 excluded from the targets for determination by the controller 610, displays a second color C2 for the measurers 500 determined as being in the approach-necessary section D1 by the controller 610, displays a third color C3 for the measurers 500 determined as being in the separation-necessary section D3 by the controller 610, and displays a fourth color C4 for the measurers 500 determined as being in the resection-possible section D2 by the controller 610. The first color C1 to the fourth color C4 may be changed in various ways within the types of colors distinguishable from each other.

In a case in which the controller 610 determines that the resection tool 3 is being incorrectly operated as will be described below, the display 620 may generate a warning signal. In this case, examples of the warning signal may be an auditory signal such as an alarm sound or a visual signal of a color different from the first color C 1 to the fourth color C4.

Hereinafter, operation states of the monitor 600 according to one embodiment of the present disclosure will be described.

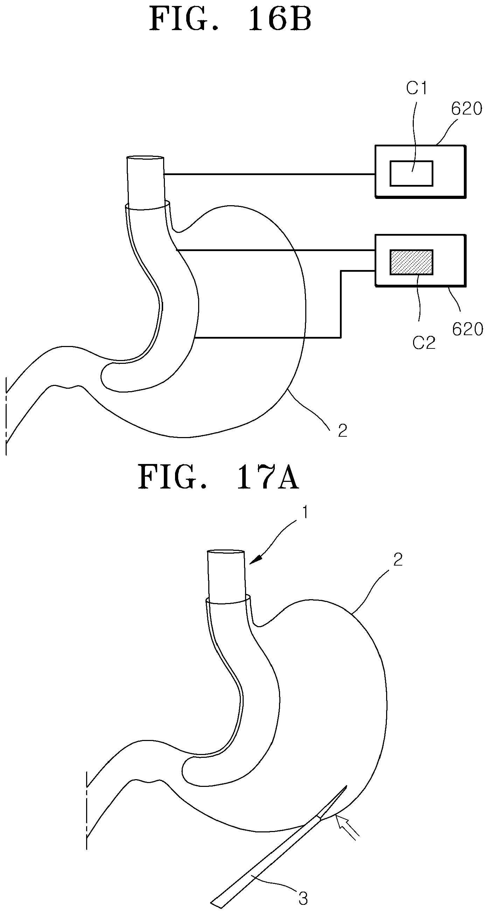

FIGS. 16A and 16B are operation diagrams schematically illustrating operation states of the controller in the process of inserting the guide.

Referring to FIGS. 16A and 16B, first, in the state in which the guide 100 is inserted into the stomach 2, the controller 610 calculates each of the number of measurers 500 disposed inside the stomach 2 and measuring the same measurement value as the initial pressure P0 inside the stomach 2 and the number of measurers 500 disposed outside the stomach 2 and measuring a different measurement value from the initial pressure P0, among the plurality of measurers 500.

Then, the controller 610 excludes the measurers 500 disposed outside the stomach 2 and measuring a different measurement value from the initial pressure P0 from targets for determination.

The controller 610 determines that the measurers 500 measuring the same measurement value as the initial pressure P0 inside the stomach 2 are in the approach-necessary section D 1.

The display 620 displays the information determined by the controller 610 to the outside. That is, as illustrated in FIG. 16, the display 620 displays the first color C1 for the measurers 500 excluded from the targets for determination by the controller 610 and displays the second color C2 for the measurers 500 determined as being in the approach-necessary section D1 by the controller 610.

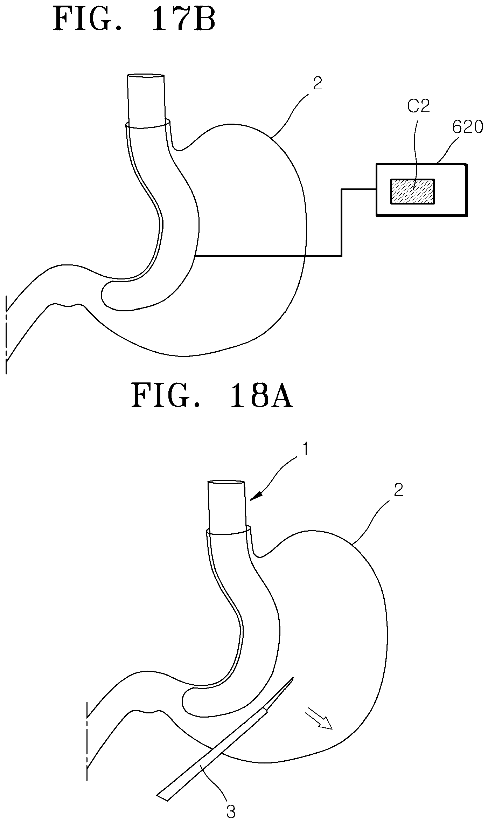

FIGS. 17A and 17B are operation diagrams schematically illustrating operation states of the controller in the approach-necessary section.

Referring to FIGS. 17A and 17B, the controller 610 determines resection position information of the resection tool 3 through a change in the pressure value measured by the measurer 500, and the display 620 displays the determined information to the outside.

More specifically, as illustrated in FIG. 17A, in a case in which the resection tool 3 presses the stomach 2 in a state in which the resection tool 3 is further away from the expander 200 than the distances from the expander 200 to the resection tool 3 that allow the user to form the remaining stomach 2 with a target size, the pressure measured by the measurer 500 is lower than the first set pressure P1.

Accordingly, the controller 610 determines that the pressure value measured by the measurer 500 is in the approach-necessary section D1 and transmits the corresponding information to the display 620. In this case, the controller 610 may use an average of pressure values measured by the remaining measurers 500 not excluded from the targets for determination among the plurality of measurers 500, that is, the measurers 500 determined to be in the approach-necessary section D1 in FIGS. 16A and 16B.

Then, by displaying the second color C2 to the outside, the display 620 visually provides information indicating that the resection tool 3 is excessively far away from the expander 200 and needs to be closer to the expander 200.

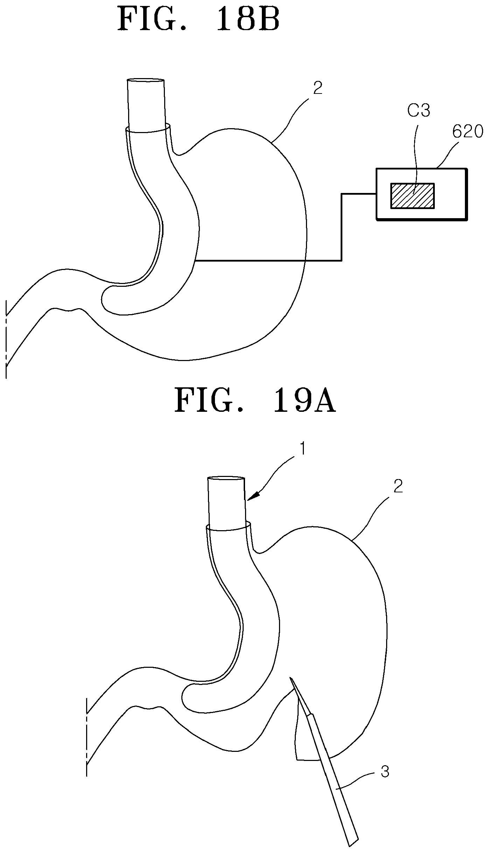

FIGS. 18A and 18B are operation diagrams schematically illustrating operation states of the controller in the separation-necessary section.

Referring to FIGS. 18A and 18B, in a case in which the resection tool 3 presses the stomach 2 in a state in which the resection tool 3 is closer to the expander 200 than the distances from the expander 200 to the resection tool 3 that allow the user to form the remaining stomach 2 with a target size, the pressure measured by the measurer 500 is higher than the second set pressure P2.

Accordingly, the controller 610 determines that the pressure value measured by the measurer 500 is in the separation-necessary section D3 and transmits the corresponding information to the display 620.

Then, by displaying the third color C3 to the outside, the display 620 visually provides information indicating that the resection tool 3 is excessively far away from the expander 200 and needs to be further from the expander 200.

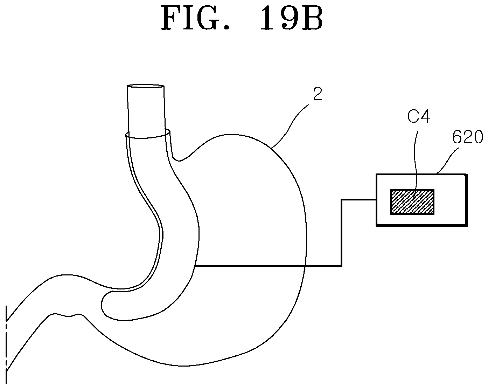

FIGS. 19A and 19B are operation diagrams schematically illustrating operation states of the controller in the resection-possible section.

Referring to FIGS. 19A and 19B, in a case in which the resection tool 3 presses the stomach 2 in a state in which the resection tool 3 is placed within the range of the distances from the expander 200 to the resection tool 3 that allow the user to form the remaining stomach 2 with a target size, the pressure value measured by the measurer 500 is between the first set pressure P1 and the second set pressure P2.

Accordingly, the controller 610 determines that the pressure value measured by the measurer 500 is in the resection-possible section D2 and transmits the corresponding information to the display 620.

Then, by displaying the fourth color C4 to the outside, the display 620 visually provides information indicating that the resection tool 3 is placed at an appropriate distance from the expander 200 and thus resection may be performed using the resection tool 3.

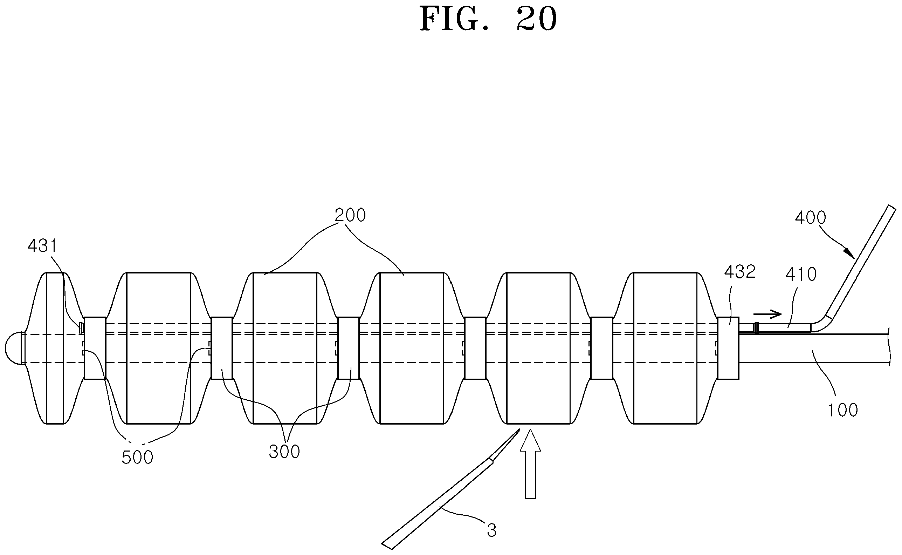

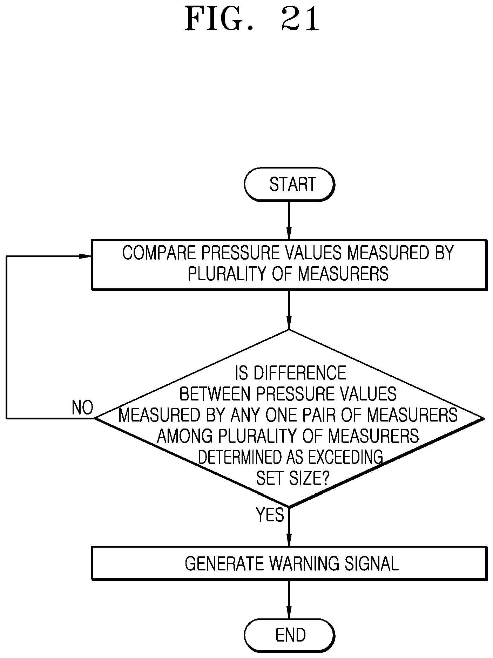

FIG. 20 is a view schematically illustrating a state in which the resection tool is incorrectly operated according to one embodiment of the present disclosure, and FIG. 21 is a flowchart schematically illustrating the process in which the monitor determines whether the resection tool is being incorrectly operated according to one embodiment of the present disclosure.

Referring to FIG. 20, the controller 610 may compare pressure values separately measured by the plurality of measurers 500 to determine whether the resection tool 3 is being incorrectly operated.

More specifically, in a case in which a local pressure is applied to any one expander 200 due to the user incorrectly operating the resection tool 3, although the average of the pressure values measured by the plurality of measurers 500 is maintained constant, a difference may occur between the pressure values measured by the corresponding measurer 500 and the measurer 500 adjacent to the corresponding measurer 500.

In a case in which the difference between the pressure values increases to a set size or more, there is a possibility that the expander 200 or the stomach 2 may be damaged due to an instantaneous pressure applied from the resection tool 3. Here, the set size of the difference between the pressure values may be changed in various ways in consideration of the strength or the like of the expander 200.

Accordingly, the controller 610 compares pressure values separately measured by the plurality of measurers 500 to determine whether a difference between pressure values measured by any one pair of measurers 500 among the plurality of measurers 500 exceeds the set size.

Then, in a case in which a difference between pressure values measured by any one pair of measurers 500 is the set size or more, the display 620 generates a warning signal to allow the user to recognize an incorrect operation of the resection tool 3.

The present disclosure has been described with reference to the embodiments illustrated in the drawings, but the embodiments are merely illustrative, and those of ordinary skill in the art should understand that various modifications and other equivalent embodiments are possible from the embodiments described herein.

Therefore, the technical scope of the present disclosure should be defined by the claims below.

## Claims

1. A sleeve gastrectomy aid device comprising:
a guide configured to be inserted into a stomach of a patient;
a plurality of expanders configured to be disposed in a longitudinal direction of the guide and have a volume that changes according to an amount of air therein;
a sealer configured to be installed at the guide and seal the plurality of expanders with each other;
an injector configured to be connected to the sealer and inject air into the expander; and
a measurer configured to be disposed inside the expander and measure a pressure value of the expander.

2. The sleeve gastrectomy aid device of claim 1, wherein the sealer includes:
a plurality of sealer bodies configured to be disposed to surround the guide and have ends of the expanders fixed thereto;
a first insertion portion configured to pass through the sealer body and have the guide inserted thereinto; and
a second insertion portion configured to pass through the sealer body and have the injector inserted thereinto.

3. The sleeve gastrectomy aid device of claim 2, wherein the sealer further includes a packing configured to be disposed to surround an outer side surface of the sealer body and bring the end of the expander in close contact with the sealer body.

4. The sleeve gastrectomy aid device of claim 1, wherein the injector is movably connected to the sealer, and a communication state between the injector and the expander is changed according to a direction of movement of the injector.

5. The sleeve gastrectomy aid device of claim 4, wherein the injector is provided as a plurality of injectors, and each of the injectors separately controls the volume of one of the expanders.

6. The sleeve gastrectomy aid device of claim 4, wherein the injector includes:
an injector body configured to be slidably connected to the sealer;
a communicator configured to pass through the injector body and have a communication state with the expander changed in accordance with movement of the injector body; and
a stopper portion configured to extend from the injector body and come in contact with the sealer to limit a movement range of the injector body.

7. The sleeve gastrectomy aid device of claim 6, wherein the stopper portion includes:
a first stopper configured to come in contact with one side surface of the sealer as the injector body moves a predetermined distance toward one side; and
a second stopper configured to be disposed to be spaced apart from the first stopper and come in contact with the other side surface of the sealer as the injector body moves a predetermined distance toward the other side.

8. The sleeve gastrectomy aid device of claim 1, further comprising a monitor configured to be connected to the measurer and provide resection position information through a pressure value measured by the measurer.

9. The sleeve gastrectomy aid device of claim 8, wherein the resection position information is information on a distance from the expander to a resection tool.

10. The sleeve gastrectomy aid device of claim 9, wherein the monitor includes:
a controller configured to determine a resection position based on the pressure value measured by the measurer; and
a display configured to display the information determined by the controller to an outside.

11. The sleeve gastrectomy aid device of claim 10, wherein the controller determines resection position sections according to a size of the pressure value measured by the measurer.

12. The sleeve gastrectomy aid device of claim 11, wherein the resection position sections include:
an approach-necessary section indicating a case in which the size of the pressure measured by the measurer is lower than or equal to a first set pressure;
a separation-necessary section indicating a case in which the size of the pressure measured by the measurer is higher than or equal to a second set pressure; and
a resection-possible section indicating that the size of the pressure measured by the measurer is between the first set pressure and the second set pressure.

13. The sleeve gastrectomy aid device of claim 11, wherein the display displays different colors according to the resection position sections.

14. The sleeve gastrectomy aid device of claim 10, wherein the measurer is provided as a plurality of measurers, and each of the measurers independently measures a pressure applied to one of the expanders.

15. The sleeve gastrectomy aid device of claim 14, wherein the controller compares pressure values measured by the plurality of measurers to determine whether the resection tool is being incorrectly operated.

16. The sleeve gastrectomy aid device of claim 15, wherein the controller determines whether a difference between the pressure values measured by any one pair of measurers among the plurality of measurers exceeds a set size.

17. The sleeve gastrectomy aid device of claim 16, wherein, in a case in which the difference between the pressure values measured by any one pair of measurers is the set size or more, the display generates a warning signal.
